Europäisches Patentamt

European Patent Office

Office européen des brevets

(19)

(11) Publication number: **0 314 421**
A1

(12) # EUROPEAN PATENT APPLICATION

(21) Application number: **88309997.0**

(22) Date of filing: **25.10.88**

(51) Int. Cl.⁴: **A 61 K 37/36**
**A 61 K 9/10**

(30) Priority: **30.10.87 GB 8725427**

(43) Date of publication of application:
**03.05.89 Bulletin 89/18**

(84) Designated Contracting States:
**AT BE CH DE ES FR GB GR IT LI LU NL SE**

(71) Applicant: **LILLY INDUSTRIES LIMITED**
**Kingsclere Road**
**Basingstoke Hants RG21 2XA (GB)**

(72) Inventor: **Bramley, Mark Richard**
**43 Brunswick Hanworth**
**Bracknell Berkshire (GB)**

**Carter, Anthony Benjamin**
**4 Lydford Close Paddock Hill**
**Frimley Surrey (GB)**

**Dunwell, David William**
**Trecairn 70 Wokingham Road**
**Crowthorne Berkshire (GB)**

(74) Representative: **Tapping, Kenneth George et al**
**Erl Wood Manor**
**Windlesham Surrey, GU20 6PH (GB)**

(54) **Somatotropin formulations.**

(57) A sustained release formulation comprises a polypeptide dispersed in an oil with a carrier and an absorption regulating agent.

EP 0 314 421 A1

Bundesdruckerei Berlin

**Description**

## SOMATOTROPIN FORMULATIONS

This invention relates to formulations useful for the sustained release of polypeptide materials, such as bovine somatotropin (BST), when injected into an animal such as a dairy cow.

It is well known that BST, when administered to dairy cows, will increase the yield of milk without adversely affecting the milk's composition. It is desirable in such use to maintain the elevated level of BST in the cow's blood stream for a prolonged period with a single application of a sustained release formulation at relatively infrequent intervals.

The present invention provides an injectable sustained release formulation of a polypeptide material, such as bovine somatotropin, which comprises an effective concentration of the polypeptide material dispersed in a carrier comprising a fatty acid salt, an absorption regulating agent and an absorbable biocompatible base consisting of a natural or synthetic oil.

The invention also provides a method of producing a prolonged release of a polypeptide material in an animal which comprises administering an effective amount of a formulation of the invention to the animal, preferably by subcutaneous injection.

In particular the invention further provides a method of increasing the milk production of a lactating cow which comprises administering an effective amount of the formulation referred to above, wherein the polypeptide material is BST, to the cow, preferably by subcutaneous injection.

The fatty acid salt is preferably the salt of a saturated or unsaturated fatty acid containing from 10 to 22 carbon atoms, the cation being preferably selected from calcium, magnesium, zinc and aluminium. The salt is preferably a salt of stearic acid, and the most preferred salt is calcium stearate.

The absorption regulating agent may be any suitable material which will control the release of the active material into the blood stream. A preferred group of materials from which the agent may be selected includes the polysaccharides, such as the dextrans, and polysaccharide derivatives, such as hydroxypropyl cellulose, hydroxypropyl methyl cellulose or ethyl cellulose; the most preferred agents are the dextrans or ethyl cellulose.

The oil is chosen from those oils which are readily obtainable in a reasonably pure form and which are physiologically acceptable and stable under the usual range of storage conditions. The oil may be a natural or synthetic oil, and may be selected for example from vegetable oils, mineral oils, fish oils and silicone oils. The preferred oils are the vegetable oils such as soybean oil, peanut oil, sesame oil, cottonseed oil, corn oil, olive oil, castor oil, palm oil, and almond oil, the most preferred being peanut oil and sesame oil.

The fatty acid salt and absorption regulating agent may be present in any ratio, and thus it may comprise substantially all fatty acid salt or all absorption regulating agent. Preferably the ratio will be in the range 1:95 to 95:1, and more preferably the ratio of fatty acid salt to absorption regulating agent will be in the range of from 1:9 to 9:1. The most preferred ratio is from 1:1 to 3:1. All references to ratios and percentages herein refer to weight, unless otherwise stated.

In order to obtain a formulation having suitable injectability and subsequent dispersability properties, it is necessary for the oil component to be present in at least a minimum amount, depending on the other components present. The optimum concentration of the oil will depend to some extent on the choice of fatty acid salt and absorption regulating agent and their relative ratios. Sufficient oil is required to ensure that the resulting formulation is in the form of an oily suspension having a viscosity which will allow ease of injection into an animal, using a hypodermic syringe, while maintaining the integrity of the subcutaneously injected mass. Thus the oil will generally be present in a minimum of 30%, and will preferably be in the range of from 30% to 80%, and preferably in the range 30% to 70%. The viscosity of the formulation will thus normally be in the range of from 2,000 to 250,000 cps, and is preferably from 10,000 to 200,000 cps, being typically about 110,000 cps.

It will be appreciated by those skilled in the art that, while entirely satisfactory formulations may be prepared containing only the above-mentioned ingredients, it may be desirable to add minor amounts of other ingredients such as dyes, preservatives, antibiotics to combat injection-site infections, viscosity-adjusting agents or stabilizers and anti-oxidants to improve the shelf-life of the formulation. The amounts of such ingredients could range up to a few percent of the final formualtion. Such additives are well-known to the formulation chemist.

The term bovine somatotropin, or BST, as used herein includes naturally occurring BST from animals and that produced by genetically modified microorganisms, and also includes known modified somatotropins bearing one or more extra amino acids on an end of the polypeptide chain.

The invention will be further described with specific reference to BST. However, it is to be understood that the invention is equally applicable to any polypeptide which it may be desirable to administer to an animal to provide a sustained concentration in the animal's bloodstream, such polypeptides having beneficial effects on, for example, growth, milk yield, health, etc. Such polypeptides may include, for example, growth-hormone releasing factor (GRF), insulin, etc.

The BST or other polypeptide may be added directly as a fine powder, such as may be produced, for example, by air milling under sterile conditions. However, it may advantageously be added in the form of microgranules, preferably having a size of less than 500 microns, being most preferably in the size range 50-500 microns. Such microgranules are prepared by well-known methods and may incorporate any suitable veterinar ally acceptable granulating agents. Among suitable granulating agents are flow agents and binders, among which may be listed, by way of example, hydrogenated castor oil, calcium stearate, hydroxypropyl cellulose and dextran.

As an example of a granulation process, the BST, which may be optionally reduced to a suitable size by air milling, may be mixed with the dry inert excipients, such as dextran or hydroxypropylcellulose (HPC) and granulated, advantageously by a dry process, preferably without heat-generation. The granulation may, for example, be carried out using dry compression, such as a standard tabletting process, followed by milling using standard means, and sieving to produce granules within the preferred range. When tabletting is to be used, a flow agent, such as calcium stearate, may advantageously be present in the tabletting composition. The granules may optionally be coated, for example with ethyl cellulose, or any suitable coating material. By suitable choice of excipients and variations in the concentration of BST in the granules, it is possible with ordinary skill to obtain granules which will disperse at different rates when used in the method and formulation of the invention. Thus it may be advantageous to incorporate in the formulation granules having different dispersion rates to provide a more even rate of BST pay-out when injected into a cow. Granulation thus can provide additional flexibility in the formulation, and additionally provides protection for the BST against the detrimental effects of heat and moisture.

When using non-granulated powdered BST, the effect of air-milling to different degrees of fineness have been found to result in the capacity of the BST to increase or decrease the viscosity of the resulting formulation, depending on the excipients included. By this process it has been found possible to prolong the pay-out while allowing a decrease in the excipients such as calcium stearate and/or dextran used in the formulation.

The formulation should contain an effective concentration of BST or other polypeptide. The concentration will depend upon several factors, such as the required dosage level, the desired payout period of the sustained release and the potency of the active material used. It is a matter which the skilled formulation chemist can readily determine once the desired parameters are set. Thus in general it has been found that an injection of from 1 to 10 grams of the formulation per dairy cow provides sustained release over a conveniently long period, that is, about 28 days, without causing discomfort. The concentration of BST in the formulation is in the range of from 5% to 45%, and is preferably from 15% to 30%, being most preferably about 25%.

The formulations of the invention are readily prepared by simply mixing the components in the desired proportions. Any suitable mixing apparatus may be used, the ingredients being added in any order and blended to give an homogeneous suspension. Thus the BST or other polypeptide may be added as a powder or in the form of microgranules to the oil in a beaker and stirred, and the remaining components added to the resulting suspension with further stirring until an homogeneous mass is obtained.

Alternatively, all the inert excipients may be mixed, in any order, and sterilised, for example by heating them in an autoclave at 120°C for 15 minutes. When cool, the BST, which may be sterilised by any suitable method known in the art, such as filtration, is added and thoroughly mixed.

If it is found necessary to incorporate a preservative, this is preferably carried out as a first step. The oil may be heated to allow the preservative to disperse. The resulting suspension is preferably allowed to cool before the BST is added.

The BST is preferably mixed in with gentle stirring. If high energy mixing is to be used, care must be taken to avoid localised heating or excessive shear. The particle size of the BST powder or microgranules should be sufficiently small to allow it to pass through the needle of the hypodermic syringe used to inject the formulation into the cow.

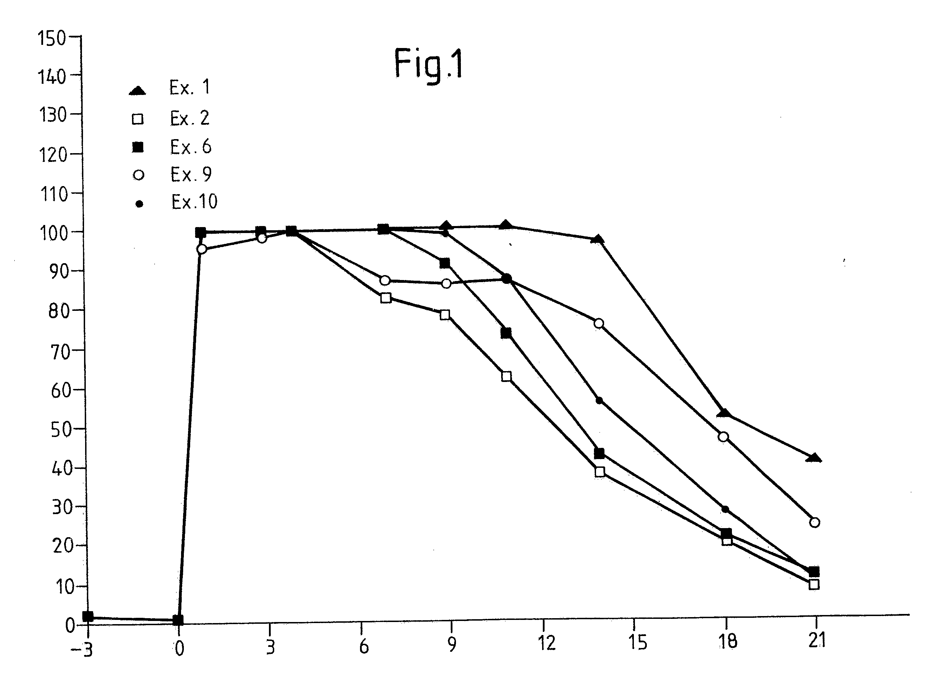

The method of carrying out the invention will now be described in more detail with reference to the following Examples and to the accompanying figures 1 to 3, in which the plasma BST levels of animals injected with formulations of the following Examples are shown over a three or four week period, as described below.

## EXAMPLE 1

Five grams of sesame oil were placed in a beaker and 1.8 gms of BST powder were added. The contents of the beaker were stirred. Calcium stearate (2.35 gms) and dextran 150 (mol. wt. 150,000) (0.85 gm) were added with further stirring until an homogeneous mass was obtained.

The resulting formulation contained, on a weight basis, 18% BST, 50% sesame oil, 8.5% dextran and 23.5% calcium stearate. The viscosity was 110,000 cps as measured by Brookfield viscometer, model no. LVT, with spindle No.4 at 1.5 r.p.m.

## EXAMPLES 2-10

The following formulations were assembled in a process similar to that used in Example 1. The figures relate to the weight of each component expressed as a percentage of the total formulation.

| EXAMPLE | Sesame Oil | Dextran | Calcium Stearate | BST |
|---|---|---|---|---|
| 2 | 50 | - | 32 | 18 |
| 3 | 50 | 32 | - | 18 |
| 4 | 50 | 5.3 | 26.7 | 18 |
| 5 | 40 | 11.2 | 30.8 | 18 |
| 6 | 50 | 16 | 16 | 18 |
| 7 | 60 | 11 | 11 | 18 |
| 8 | 30 | 13.9 | 38.1 | 18 |
| 9 | 30 | 26 | 26 | 18 |
| 10 | 40 | 21 | 21 | 18 |

EXAMPLE 11

In order to assess the effectiveness of the invention in sustaining the release of BST, samples of formulations made in the above Examples were evaluated in vivo in sheep. Each Example was tested in 4 sheep, which were injected subcutaneously behind the shoulder with 2 ml of the formulation, containing about 320 mg of BST. Blood samples (approx. 5 ml) were taken at intervals by jugular venipuncture, and placed in tubes containing heparin. The plasma was separated using a centrifuge, and the concentration of BST determined by double antibody radioimmunoassay.

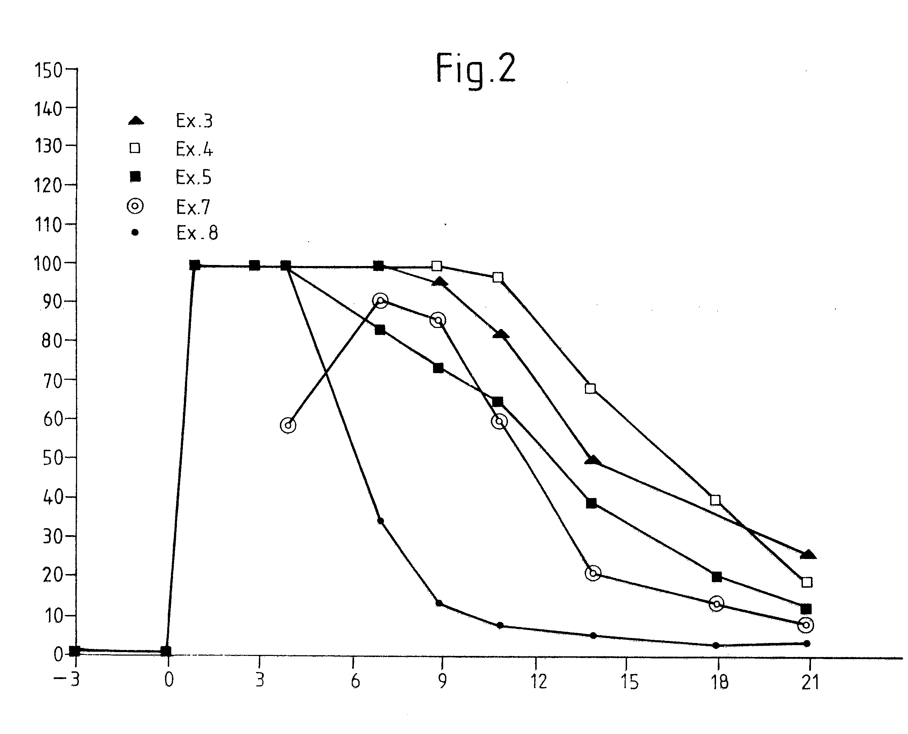

The plasma BST levels averaged over the 4 sheep are plotted over a period of 24 days, from 3 days before injection, in figures 1 and 2. It can be seen that BST levels remain well above the initial level, as measured before injection of the formulation over the whole of this period.

EXAMPLE 12

Further batches of the product of Example 1 were obtained, each batch using 5 times the quantities of materials used in Example 1. The sesame oil, calcium stearate and dextran was mixed until homogeneous and sterilised at 120°C for 15 minutes in an autoclave. The BST, sterilised by filtration, was added to the cooled excipients and thoroughly mixed in.

Tests were carried out on beef steers to assess the effective duration of the treatment. All animals used in the test were issued the same amount of feed each, twice daily. Water was available ad libitum throughout the period. Urine samples were collected and the urea nitrogen excretion levels monitored. A decrease in the urea nitrogen level is taken as a measure of the increased use of amino acids in the production of protein due to the effect of the somatotropin.

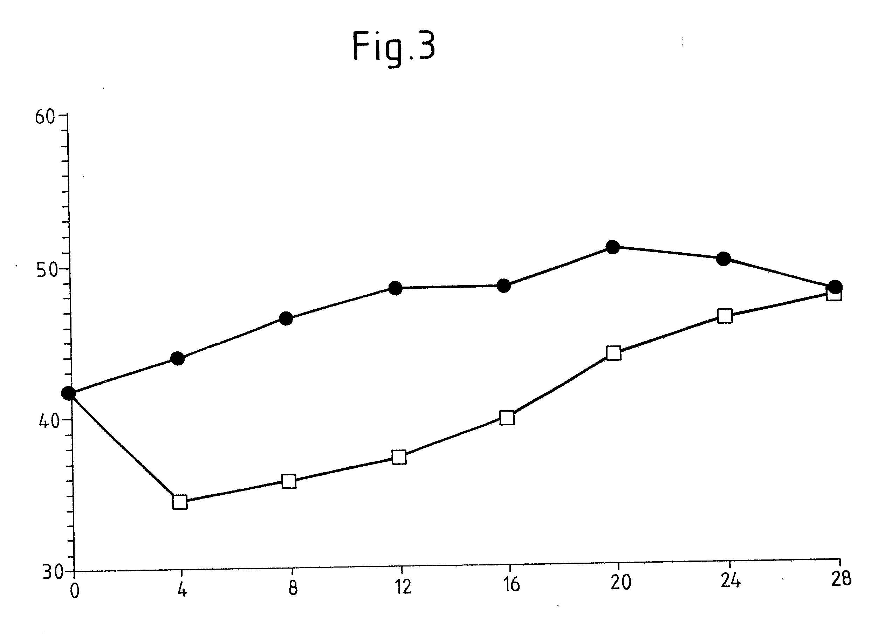

The results of the tests are shown in figure 3. Nine animals were each treated by subcutaneous injection of 960 mgm of BST, using the formulation described above. The results (open squares in figure 3) show sustained suppression of urea nitrogen in the urine of the test steers when compared with untreated control animals (closed circles), over a period approaching 28 days.

In the following Examples, the formulations were prepared using the method of Example 12. Where microgranules were used, these were prepared by tabletting the stated ingredients and grinding the tablets to provide granules within the range 100-425 microns.

| Example No. | Component | % |
|---|---|---|
| 13 | BST | 25 |
| | Calcium stearate | 8 |
| | Dextran 150 | 8 |
| | Sesame oil | 59 |
| | | |
| 14 | BST | 18 |
| | Calcium stearate | 10 |
| | Silica R974 | 4 |
| | Sesame oil | 68 |
| | | |
| 15 | BST (microgranules) | 35 |
| | Calcium stearate | 10 |
| | Dextran 9.4 | 5 |
| | Sesame oil | 50 |

BST microgranules contained

| | | |
|---|---|---|
| | BST | 50 |
| | Calcium stearate | 3 |
| | HPC | 47 |

| Example No. | Component | % |
|---|---|---|

16        As 15, but dextran 9.4 was substituted for HPC in the microgranules

17        As 15, but ethyl cellulose was substituted for HPC in the microgranules

18-20    As 15-17, but the granules were coated with 1% ethyl cellulose

21

| BST microgranules | 35 |
|---|---|
| Calcium stearate | 5 |
| Dextran 9.4 | 5 |
| Silica R974 | 2 |
| Sesame oil | 53 |

BST microgranules as used in Example 15.

22

| BST microgranules | 35 |
|---|---|
| Calcium stearate | 10 |
| Dextran 150 | 5 |
| Sesame oil | 50 |

BST microgranules as used in Example 16

23

| BST microgranules | 35 |
|---|---|
| Calcium stearate | 8 |
| Dextran 9.4 | 5 |
| Silica R974 | 2 |
| Sesame oil | 50 |

BST microgranules as used in Example 17

24

| BST | 18 |
|---|---|
| Calcium Stearate | 8.8 |
| Dextran 150 | 3.2 |
| Silica R974 | 2 |
| Sesame oil | 68 |

## Claims

An injectable sustained release formulation of a polypeptide material comprising an effective concentration of the polypeptide material dispersed in a carrier comprising an absorbable biocompatible base consisting of a natural or synthetic oil and at least one component selected from a fatty acid salt and an absorption regulating agent

2. A formulation of claim 1 wherein the polypeptide material is bovine somatotropin.

3. A formulation of claim 1 or claim 2 comprising both a fatty acid salt and an absorption regulating agent.

4. A formualtion of any one of claims 1 to 3 wherein the formulation has a viscosity in the range of from 2,000 to 250,000 cps.

5. A formulation of claim 4 wherein the viscosity is in the range of from 10,000 to 200,000 cps.

6. A formulation of any one of claims 1 to 5 wherein the fatty acid salt is calcium stearate.

7. A formulation of any one of claims 1 to 6 wherein the absorption regulating agent is dextran.

8. A formulation of any one of claims 1 to 7 wherein the oil is sesame oil.

9. A formulation of any one of claims 1 to 8 wherein the ratio of fatty acid salt to absorption regulating agent is in the range 1:95 to 95:1.

10. A formulation of claim 8 wherein the ratio is in the range 1:9 to 9:1.

11. A formulation of claim 10 wherein the ratio is from 1:1 to 3:1.

12. A formulation of any one of claims 1 to 11 which comprises at least 30% by weight of the oil.

13. A formulation of any one of claims 1 to 12 wherein the polypeptide material is present in the form of microgranules.

14. A formulation of claim 13 wherein the microgranules are in the size range of from 50-500 microns.

15. A formulation of any one of claims 1 to 14 wherein the bovine somatotropin comprises from 5 to 45% by weight of the formulation.

16. A formulation of claim 15 wherein the weight range is 15-30%.

17. A method of increasing the milk production of a lactating cow which comprises administering an effective amount of a formulation as claimed in any one of claims 1 to 16.

## Claims for the following Contracting State: ES

1. A process for preparing an injectable sustained release formulation of a polypeptide material comprising dispersing an effective concentration of the polypeptide material in a carrier comprising an absorbable biocompatible base consisting of a natural or synthetic oil, and including in the dispersion at least one component selected from a fatty acid salt and an absorption regulating agent.

2. A process of claim 1 wherein the polypeptide material is bovine somatotropin.

3. A process of claim 1 or claim 2 including both a fatty acid salt and an absorption regulating agent.

4. A process of any one of claims 1 to 3 wherein the excipients are mixed together before the polypeptide material is added.

5. A process of any one of claims 1 to 4 wherein the fatty acid salt is calcium stearate.

6. A process of any one of claims 1 to 5 wherein the absorption regulating agent is dextran.

7. A process of any one of claims 1 to 6 wherein the oil is sesame oil.

8. A process of any one of claims 1 to 7 wherein the ratio of fatty acid salt to absorption regulating agent is in the range of from 1:9 to 9:1.

9. A process of any one of claims 1 to 8 wherein the oil comprises at least 30% by weight of the formulation.

10. A process of any one of claims 1 to 9 wherein the polypeptide material comprises from 5 to 45% by weight of the formulation.

11. A method of increasing the milk production of a lactating cow which comprises administering an effective amount of a formulation prepared by a process of any one of claims 1 to 10.

Fig.1

Ex. 1 ◄
Ex. 2 □
Ex. 6 ■
Ex. 9 ○
Ex.10 ●

Fig.2

Ex.3 ◄
Ex.4 ☐
Ex.5 ■
Ex.7 ◎
Ex.8 ●

# Fig.3

EP 0 314 421 A1

European Patent Office

**PARTIAL EUROPEAN SEARCH REPORT**
which under Rule 45 of the European Patent Convention
shall be considered, for the purposes of subsequent
proceedings, as the European search report

Application number

EP 88 30 9997

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 211 691 (ELI LILLY CO.)<br>* Column 1, line 18 - column 3, line 16; column 4, lines 1-17 * | 1-4,8, 15,16 | A 61 K 37/36<br>A 61 K 9/10 |
| Y | | 5-7,9-11,13, 14 | |
| | -- | | |
| X | EP-A-0 140 255 (SUMITOMO CHEMICAL CO. LTD.)<br>* Page 6, line 14 - page 7, line 10; claims 1-14 * | 1,8,12 | |
| Y | | 5-7,9-11,13, 14 | |
| | -- | | |
| Y | GB-A-1 155 036 (ETHAN ALLAN BROWN)<br>* Claims 1-14 * | 5-7,9-11,13, 14 | TECHNICAL FIELDS SEARCHED (Int. Cl.4) |
| | -- | | A 61 K |
| | ./. | | |

### INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely: 1-16
Claims searched incompletely: 17
Claims not searched:
Reason for the limitation of the search:

Method for treatment of the human
or animal body by surgery or therapy
(See art. 52(4) of the European
Patent Convention)

| Place of search | Date of completion of the search | Examiner |
|---|---|---|
| The Hague | 09-02-1989 | TZSCHOPPE |

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| P,Y | EP-A-0 257 368 (AMERICAN CYANAMID CO.)<br><br>* Page 1, line 1 - page 4, line 28 * | 5-7,9-11,13,14 | |
| X | EP-A-0 213 851 (ELI LILLY AND CO.)<br><br>* Column 1, line 29 - column 4, line 35 * | 1,8,12 | |
| X | EP-A-0 177 478 (MONSANTO CO.)<br><br>* Page 13, line 29 - page 14, line 13; claims 1-41 * | 1,2,4,6,8,15 | TECHNICAL FIELDS SEARCHED (Int Cl 4) |
| A | EP-A-0 211 601 (INTERNATIONAL MINERALS AND CHEMICAL CO.)<br><br>* Page 6, line 31 - page 7, line 9; page 8, lines 10-21; claims 1-14 * | | |
| A | EP-A-0 193 917 (AMERICAN CYANAMID CO.)<br><br>* Calims 1-24 * | | |

-------------

EPO Form 1505.3  06.78